# EUROPÄISCHE PATENTSCHRIFT

(11) **EP 1 352 081 B1**
(45) Veröffentlichungstag und Bekanntmachung des Hinweises auf die Patenterteilung: **22.04.2009**
(21) Anmeldenummer: 02716066.2
(22) Anmeldetag: 08.01.2002
(51) Int. Cl.: C12Q 1/00

(54) **VERFAHREN UND VORRICHTUNG ZUM NACHWEIS VON MIKROORGANISMEN AUF OBERFLÄCHEN**
METHOD AND DEVICE FOR DETECTING MICROORGANISMS ON SURFACES
PROCEDE ET DISPOSITIF DE DETECTION DE MICRO-ORGANISMES SUR DES SURFACES

(30) Priorität: 09.01.2001 DE 10100581
(43) Veröffentlichungstag der Anmeldung: 15.10.2003
(73) Patentinhaber: Schies, Ursula, 81241 München (DE); Theissen, Hubert, 12203 Berlin (DE)
(72) Erfinder: Schies, Ursula, 81241 München (DE); Theissen, Hubert, 12203 Berlin (DE)
(74) Vertreter: TBK-Patent
(86) Internationale Anmeldenummer: PCT/EP2002/000113
(87) Internationale Veröffentlichungsnummer: WO 2002/055729

(56) Entgegenhaltungen:
- EP-A- 0 193 385
- EP-A- 0 433 053
- EP-A- 0 751 393
- WO-A-00/55357
- WO-A-00/79235
- WO-A-92/16648
- WO-A-94/21816
- US-A- 5 474 910

## Beschreibung

Die Erfindung betrifft ein Verfahren zum Nachweis von Mikroorganismen auf Oberflächen.

Mikroorganismen können gesundheitsgefährdende Auswirkungen auf Menschen ausüben, wenn sie in der Umgebungsluft vorhanden sind und auf den menschlichen Organismus einwirken. Besonders in der Arbeitswelt machen sich zunehmend bakteriologische oder virologische Erkrankungen bemerkbar, die nicht nur gesundheitliche, sondern auch volkswirtschaftliche Schäden in hohem Ausmaß verursachen. Eine Ursache derartigen Eintrags von Mikroorganismen in die Umgebungsluft wird Lüftungs- und Klimaanlagen zugeschrieben, auf deren Filtern sich die. Mikroorganismen festsetzen können.

Mikroorganismen üben ferner gesundheitsschädliche Einflüsse auf den menschlichen Organismus aus, wenn sie auf Wandoberflächen in Wohnungen oder anderen Innenräumen, aber auch auf Mauerwerk im Außenbereich eingewachsen sind. Gerade in Mauerwerk führt übermässiger Mikroorganismenbewuchs zu Verfall und Zerstörung ganzer Bauwerke. Außer Mauerwerk können Mikroorganismen auch auf Wandoberflächen aus Holz oder Metall, gerade in Industrieanlagen wachsen, wobei die geschilderten Nachteile ebenso eintreten

In WO-A-00 79235 wird der Nachweis von Substanzen, z.B. von Pilzen, durch elektromagnetische Strahlung beschrieben.

WO-A-94 21816 beschäftigt sich mit Verfahren und Reagenziensätzen für den Nachweis von Mikroorganismen, wobei ein Enzymsystem verwendet wird, das eine Spaltungsreaktion katalysiert.

US-A-5 474 910 beschäftigt sich mit einem Verfahren und einer Vorrichtung für den Nachweis von fluoreszierenden Mikroorganismen auf einer Fläche oder in einem Raum.

WO-A-00 55357 offenbart Nachweisvorrichtungen, in denen das Wachstum von Mikroorganismen auf einem Substrat mit antimikrobiellen Mitteln nachgewiesen wird.

EP-A-0 193 385 bezieht sich auf ein Gerät und ein Verfahren für mikrobiologische Untersuchungen, wobei das Wachstum von Mikroororganismen in einem Teststreifen mit einer Kamera unter Lichtbestrahlung nachgewiesen wird.

WO-A-92 16648 beschreibt eine Chemilumineszenz-Untersuchung für den Nachweis und die Charakterisierung von Mikroorganismen durch Lichtabgabe.

In EP-A-0 433 053 wird ein Verfahren und eine Vorrichtung für den schnellen Nachweis der Effektivität eine Sterilisationsvorganges beschrieben, wobei ein Indikatormittel Ninhydrin sein kann.

In EP-A-0 816 513 wird ein Verfahren zum Nachweis von Mikroorganismen auf Oberflächen beschrieben, wobei ein Klebestreifen eingesetzt wird.

Es ist daher die Aufgabe der Erfindung, ein neues, vereinfachtes Verfahren zum Nachweis von Mikroorganismen auf Oberflächen bereitzustellen, mit dem es möglich ist, die Mikroorganismen einfach sichtbar zu machen und dadurch ein Entscheidungskriterium zu schaffen, diese Oberflächen auszutauschen, bzw. von den Mikroorganismen zu befreien.

Diese Aufgabe wird erfindungsgemäß durch ein Verfahren zum Nachweis von Mikroorganismen auf Oberflächen gelöst, bei dem der Mikroorganismus mit einer Lichtquelle bestrahlt wird, die eine Wellenlänge beinhaltet, die bei Einstrahlung auf den Mikroorganismus eine Photodetektion des Mikroorganismus zulässt und der Mikroorganismus durch eine Farb- oder Lichtreaktion nachgewiesen wird. Die Mikroorganismen können vorzugsweise Pilze sein.

Unter dem Begriff "Photodetektion" soll ein Nachweis durch Absorption des eingestrahlten Lichts, aber auch durch eine Anregung von Komponenten oder Strukturen des Mikroorganismus durch das eingestrahlte Licht unter Emission einer Strahlung mit charakteristischer, nachzuweisender Wellenlänge verstanden werden.

Vorzugsweise beinhaltet die Lichtquelle ultraviolettes Licht, insbesondere einer Wellenlänge im Bereich von 210 bis 310 nm.

Gemäß der Erfindung wird der Mikroorganismus zusätzlich zu der Bestrahlung unter Photodetektion ferner einer Farbreaktion unterzogen. Die-Farbreaktion kann insbesondere chemisch, vorzugsweise durch Ninhydrin vermittelt sein.

Die Farb- oder Lichtreaktion kann alternativ durch eine immunologische Reaktion, bevorzugt durch eine Antigen-Antikörper-Reaktion, beispielsweise unter Einschluss von Biolumineszenz vermittelt sein. Dabei kann die Farb- oder Lichtreaktion durch Reaktion einer chromophoren oder fluorophoren Substanz mit dem Mikroorganismus oder im Falle der immunologischen Reaktion mit einem Enzym erfolgen.

Auf die zu untersuchende Fläche kann beispielsweise eine Ninhydrin-Lösung in einem oder mehreren geeigneten Lösungsmitteln, vorzugsweise leichtflüchtigen Lösungsmittel aufgebracht werden. Bevorzugt wird die so behandelte Oberfläche erhitzt, insbesondere auf einen Temperaturbereich von 70°C bis 100°C. Das Erhitzen kann beispielsweise mit einem Föhn und/oder Heizstrahler erfolgen.

Im Verlauf der Farb- oder Lichtreaktion kann die zu untersuchende Oberfläche mit einem mildwirkenden oberflächenaktiven Mittel gereinigt werden. Insbesondere kann die Oberfläche durch eine Proteinlösung gesättigt werden.

Der Auftrag eines Reagens zum Nachweis des Mikroorganismus kann vor Installation der Oberfläche in der Endanwendung oder erst zum Zeitpunkt der Kontrolle erfolgen.

Gemäß einer insbesondere bevorzugten Ausführungsform der Erfindung umfasst das Verfahren folgende Schritte:
a) Auftragen einer mit Mikroorganismen reaktiven Substanz auf die zu untersuchende Oberfläche,
b) Durchführen einer Reaktion der Mikroorganismen mit der reaktiven Substanz unter Bildung eines Reaktionsproduktes, und
c) Sichtbarmachen des Reaktionsprodukts.

Weiter bevorzugt ist ein Verfahren bei dem der Nachweis der Mikroorganismen mittelbar erfolgt, wobei von der zu untersuchende Oberfläche ein Folienabzug oder Filmabzug hergestellt wird, vorzugsweise unter Verwendung eines Polyacetatfilms. Der Folien- oder Filmabzug kann insbesondere durch Sprühen und/oder mit einem Schwamm hergestellt werden. Dabei können die Mikroorganismen auf dieser Folie bzw. auf dem Film nachgewiesen werden.

Gemäß einer weiteren bevorzugten Ausführungsform der Erfindung wird die Photodetektion und/oder Farbreaktion mit einer Kamera in regelmäßigen oder unregelmäßigen Abständen verfolgt. Die Detektion kann computerunterstützt, insbesondere durch Ferndiagnose durchgeführt werden. Hierbei können Telekommunikationseinrichtungen, wie Datenleitungen, z.B. eine computergestützte Internetverbindung genutzt werden.

Erfindungsgemäß wird ferner eine Vorrichtung zum Nachweis von Mikroorganismen zur Verfügung gestellt, die folgendes .umfasst:
eine Lichtquelle zur Bestrahlung eines Mikroorganismus auf Oberflächen, die eine Wellenlänge beinhaltet, die bei Einstrahlung auf den Mikroorganismus, eine Photodetektion des Mikroorganismus zulässt und
eine Vorrichtung zum Auftrag eines Reagens auf die Oberfläche, welches eine Farb- oder Lichtreaktion zum Nachweis des Mikroorganismus ermöglicht,
eine Detektionseinrichtung zur Erkennung des Bewuchses des Mikroorganismus, und
eine Diskriminierungseinrichtung zur Bewertung des Grades des Mikroorganismusbewuchses.

Die Detektionseinrichtung ist vorzugsweise eine digitale Kamera, z.B. eine CCD-Kamera. Die Diskriminierungseinrichtung ist vorzugsweise ein Computer, der eine Subtraktion mehrerer von der digitalen Kamera aufgenommener Bilder der zu untersuchenden Oberfläche durchführt. Auf Grundlage der Berechnung der Diskriminierungseinrichtung kann ein Warnsignal ausgelöst werden, sofern der Grad des Mikroorganismusbewuchses einen kritischen Grenzwert übersteigt. Die Überwachung kann insbesondere durch ein Fernwartungssystem, vorzugsweise über Telekommunikationseinrichtungen gesteuert werden.

Speziell kann das erfindungsgemässe Verfahren und die erfindungsgemässe Vorrichtung zur Kontrolle von Filtern, insbesondere in Klima- oder Lüftungsanlagen eingesetzt werden. Besonders geeignet ist es für Anlagen zur Atemluftversorgung, allgemein auch als Schutzbelüftungsanlagen bekannt. Derartige Schutzbelüftungsanlagen werden beispielsweise in Kabinen auf Erdbaumaschinen oder in Flurförderzeugen verwendet.

Es ist ferner bevorzugt, daß die Filterrückseite mit infrarotem, sichtbarem oder ultraviolettem Licht bestrahlt wird. Zusätzlich kann die Filteroberfläche auf der Reinraumseite mit einer chromo- oder fluorophoren Chemikalie behandelt werden. Alternativ kann die Filteroberfläche auf der Reinraumseite mit Chemikalien imprägniert werden, die nach Biotransformation chromo- oder fluorophor werden. Die Auswertung kann durch Sichtinspektion und/oder durch Computerauswertung erfolgen.

Andererseits kann das erfindungsgemässe Verfahren und die erfindungsgemässe Vorrichtung auch zur Überwachung von Mikroorganismusbewuchs auf Wandoberflächen wie beispielsweise Mauerwerk oder auch auf einer Tapete, auf einer Fläche eines Möbelstücks, auf Holzoberflächen, z.B. Fachwerk oder auf einer Metalloberfläche dienen.

In der vorliegenden Erfindung soll der Ausdruck "Mikroorganismus" bzw. "Mikroorganismen" nicht nur den Mikroorganismus selbst, sondern auch Bestandteile von Mikroorganismen und/oder Produkte dieser Mikroorganismen beinhalten. Als Mikroorganismen können erfindungsgemäß Pilze, aber auch Bakterien, Cyanobakterien, Protozoen, Schleimpilze, niedrige Algen und Metazoen und ferner Viren und Phagen genannt werden. Darunter fallen auch Symbiosen aus diesen Mikroorganismen, bspw. aus Algen und Pilzen, welche die Flechten einschließt.

Die Erfindung und die ihr zugrundeliegende Problematik wird anhand der folgenden Ausführungsbeispiele näher beschrieben.

Es sei betont, dass die Erfindung nicht auf diese Beispiele beschränkt ist, sondern durch diese lediglich beispielhaft erklärt wird. Es sind vielfältige Abwandlungen vorstellbar, solange sie sich in dem durch die folgenden Patentansprüche definierten Schutzumfang der Erfindung bewegen.

### Überwachung von Filteranlagen

Die Filter in Lüftungs- und Klimaanlagen dienen dazu, Partikel und Keime aus der umgewälzten Luft zu entfernen, die entweder die Gesundheit gefährden oder für die in den jeweiligen Räumen ablaufenden Arbeitsprozesse schädlich sind. Die ordnungsgemäße Funktion solcher Filter kann durch die entsprechende Nutzung stark beeinträchtigt werden. Dazu zählen neben mechanischen Beschädigungen vor allem der Befall durch Mikroorganismen. Im Besonderen ist hier der Bewuchs mit Pilzen zu nennen, da das Pilzmycel in der Lage ist, die einzelnen Filterschichten zu durchwachsen und es dann auf der Reinluftseite zur Freisetzung von ggfs. die Gesundheit oder den Produktionsprozeß beeinträchtigenden Pilzsporen kommt. Deshalb ist es erforderlich, die Filtergüte zu überwachen, um so rechtzeitig einen Austausch durchführen zu können.

Bisher wird diese technische Überprüfung in regelmäßigen Abständen durch Überwachung der Druckdifferenz und der Betriebszeit durchgeführt. Weiterhin ist der optische Eindruck entscheidend. Das Durchwachsen von Pilzen kann bisher höchstens durch hygienische Untersuchungen festgestellt werden. Damit kann ein Durchschlagen der Filter frühestens beim nächsten Wartungsintervall entdeckt werden, wobei bei einer normalen Sichtkontrolle lediglich bereits sporulierende Schimmelpilze erkannt werden. Einzelne Pilzhyphen sind mit bloßem Auge nur schwer zu erkennen. Dies hat zur Folge, daß die Filter entweder prophylaktisch bei den Wartungen ausgewechselt werden, obwohl sie noch im vollen Umfang funktionsfähig sind, oder daß es nach Ausfall der Funktion bis zur nächsten Wartung zum Eintrag von Schadpartikeln oder -keimen in der Raumluft kommt.

Gemäß einer ersten bevorzugten Ausführungsform der Erfindung wird das Verfahren zur Detektion von Mikroorganismen auf Filteroberflächen angewandt, womit eine ökonomische und zeitnahe Überwachung des Austauschzeitpunkts der Filter in den Filteranlagen ermöglicht wird.

Das nachfolgend beschriebene erfindungsgemäße Verfahren erlaubt die automatische Überwachung der Filterfunktion bspw. in Lüftungs- und Klimaanlagen. Filtermaterialien haben im allgemeinen eine sehr homogene Oberfläche, um über ihre gesamte Fläche eine gleichbleibende Wirkung zu erzielen. Beeinträchtigungen des Filters zeigen sich deshalb zuerst in einer Veränderung der Oberfläche an einzelnen Stellen. Für einige Anwendungen werden auch Filter mit unebener oder welliger Oberfläche auf der Reinraumseite eingesetzt. In diesem Fall kann über die unebene oder wellige Oberfläche ein weiterer Filter wie beispielsweise ein Filtertuch mit homogener Oberfläche aufgebracht werden, auf der die Nachweisreaktion stattfindet. Eine Kontrolle der Filtergüte kann also in der optischen Überwachung der oberflächigen Homogenität der letzten bzw. sichtbaren Filterschicht bestehen. Zu diesem Zweck wird in die Rückwand des Filtergehäuses eine Lichtquelle eingebaut. Besonders geeignet dafür sind Quellen für UV-Licht, beispielsweise auch Laserlichtquellen, insbesondere der Wellenlängen 210-310 nm, vorzugsweise 280 nm, da biologisches Material, wie z.B. ein Pilzmycel, UV-Strahlung dieser Wellenlängen stark absorbiert und dadurch als dunkle Muster erkennbar wird.

Zusätzlich kann die letzte Filteroberfläche vorzugsweise mit einer chemischen Substanz behandelt werden, um dadurch eine Farbreaktion zwischen Mikroorganismus und dieser Substanz hervorzurufen und dadurch den Kontrast zu verbessern.

Eine weitere Möglichkeit besteht in der Imprägnierung mit Chemikalien, die durch Biotransformation in fluoreszierende oder phosphoreszierende Substanzen umgewandelt werden, um dadurch ein Farb- oder Lichtsignal zu erzeugen (z.B. durch Biolumineszenz) und so das gewünschte Signal zu verstärken.

Die Detektion der optischen Veränderung kann durch einfache Beobachtung, aber auch mit Hilfe einer Kamera, vorzugsweise einer CCD-Kamera erfolgen. Beim Einsatz einer CCD-Kamera wird das aufgenommene Bild digitalisiert und einem Computer zugeführt. Der Computer führt eine rechnerische Subtraktion von letztem Bild und ursprünglichem Bild durch. Die dabei erhaltenen Differenzen werden in eine Kennzahl umgerechnet. Optional können auch mehrere Bilder aufgenommen werden.

Eine weitere Analyse der bereits ermittelten Kennzahlen auf einen erkennbaren Trend hin führt dann gegebenenfalls zur Auslösung eines Warnsignals. Der Einsatz eines Computers erlaubt dabei neben der Auslösung optischer und/oder akustischer Warnungen auch die Weitergabe des Signals über Fernwartungssysteme. Solche Fernwartungssysteme eignen sich insbesondere zur Ferndiagnose mittels Telekommunikation, z.B. Fernabfrage über das Internet. Auch der Einsatz protokollierender Systeme (z.B. Drucker) ist möglich. Die Intervalle, in denen ein Bild aufgenommen wird, kann in weiten Bereichen an die Sicherheitsbedürfnisse angepaßt werden.

### Verfahren zum Nachweis von Pilzbefall im Mauerwerk

Im Folgenden wird eine weitere bevorzugte Ausführungsform der Erfindung unter Bezugnahme auf eine vorteilhafte Anwendung des erfindungsgemäßen Verfahren beschrieben. Der Pilzbefall von Mauerwerk in Innenräumen und im Außenbereich stellt nicht nur ein Gesundheitsproblem, sondern auch eine Gefahr durch Zersetzung des Mauerwerks durch die Mikroorganismen dar, was die Baufälligkeit des gesamten Gebäudes oder seiner tragenden Wände erhöhen kann.

Die Behandlung von mit Pilzen befallenem Mauerwerk besteht im allgemeinen im Entfernen des kontaminierten Materials. Dabei wird das Material so weit entfernt, bis keinerlei Befall mehr sichtbar ist. Allerdings bleibt dabei der Befall unberücksichtigt, der optisch nicht erkennbar ist. Dadurch kann es nach kurzer Zeit zu einer erneuten Ausweitung des Mikroorganismenbewuchses kommen.

Die nachstehend beschriebenen Techniken bieten die Möglichkeit, auch kleine Restmengen an Mikroorganismen (oft handelt es sich um Pilzmycel) in der Mauer- oder Putzmatrix nachzuweisen. Bevorzugt wird zu diesem Nachweis eine Farbreaktion eingesetzt. Eine besonders geeignete Detektion beinhaltet die allgemein bekannte Ninhydrin-Färbung.

### Ninhydrin-Färbung

Ein Verfahren ist die Färbung von Proteinen mit Ninhydrin. Bei der Reaktion mit Ninhydrin (Indan-1,2,3-trion) werden die Aminosäuren oxidativ desaminiert und decarboxyliert. Dabei entstehen die um ein Kohlenstoffatom ärmeren Aldehyde. Reduziertes Ninhydrin kondensiert mit abgespaltenem Ammoniak und unverändertem Ninhydrin zu einem rot- bis blauvioletten Farbstoff.

Die erfindungsgemäße Durchführung des Nachweises kann vorzugsweise auf zwei verschiedene Arten erfolgen: Die Durchführung ist aber nicht hierauf beschränkt.
- Auf die untersuchende Fläche wird beispielsweise aus einer Sprühflasche eine Ninhydrinlösung (geeigneter Weise 2,5 g/l in 2-Propanol) aufgebracht. Anschließend wird die Fläche gleichmäßig auf etwa 80°C erwärmt. Hierfür kann ein Heißluftföhn und/oder ein Heizstrahler verwendet werden. Bei Vorhandensein von Proteinen aus dem Mikroorganismus bildet sich eine blaue Färbung.
- Auf die zu untersuchende Fläche wird beispielsweise aus einer Sprühdose ein Film, z.B. ein Klarsichtfilm, der bevorzugt aus Polyacetat bestehen kann, aufgesprüht und nach einer ausreichenden Aushärtzeit abgezogen. Der Nachweis der Proteine auf dem Film erfolgt dann wie oben beschrieben. Das Verfahren ist einfach und leicht durchzuführen.

### Antikörperreaktion

Eine weitere bevorzugte Möglichkeit der Detektion besteht in einer Antikörperreaktion. Hierbei kann eine Antikörperfärbung über eine enzymvermittelte Farbreaktion oder auch eine Antikörper-Lumineszenzreaktion vorteilhaft eingesetzt werden. Als Ausführungsbeispiel wird hier eine Antikörperfärbung beschrieben.

Die Oberflächen aller lebenden Zellen tragen Substanzen (Oberflächenantigene), gegen die sich in Tieren Antikörper bilden lassen. Diese Antikörper binden sehr spezifisch an die Oberflächenantigene und der so gebildete Komplex läßt sich mit Hilfe eines zweiten Antikörpers, der gegen den ersten gerichtet ist und an den ein Enzym gekoppelt ist, über eine enzymvermittelte Farbreaktion nachweisen.

Eine derartige enzymvermittelte Antikörperfärbung kann vorzugsweise erfolgen, indem ein für den zu untersuchenden Mikroorganismus spezifischer Antikörper eingesetzt wird. Man gibt nun einen zweiten Antikörper (sekundärer Antikörper) hinzu, der seinerseits spezifisch an den primären Antikörper bindet und an den chemisch ein Enzym, z.B. Peroxidase aus Meerrettich oder Alkalische Phosphatase gekoppelt wurde. So bildet sich an der Stelle des Mikroorganismenbefalls ein Komplex, der durch das gebundene Enzym in der Lage ist, eine spezifische Farbreaktion (z.B. die durch Alkalische Phosphatase katalysierte Reaktion von farblosem para-Nitrophenylphosphat zu violettem para-Nitrophenolat) durchzuführen.

Das Hauptproblem dabei besteht darin, die unspezifische Adhäsion des primären und des sekundären Antikörpers an die Wandoberfläche, Mauer- bzw. Putzoberfläche zu unterbinden.

Die zu untersuchende Oberfläche kann in einem ersten Schritt mit einem schwachen Detergenz, z.B. Tween^{®} 20 abgewaschen werden. Anschließend kann sie mit einer verdünnten Proteinlösung, z.B. Rinderserumalbumin, die ebenfalls ein schwaches Detergenz enthält, behandelt werden. Nach einem weiteren Waschschritt wird die verdünnte Lösung des primären Antikörpers aufgebracht, so dass er mit dem auf der Oberfläche haftenden Mikroorganismus reagieren kann. Die Oberfläche wird nochmals gewaschen, vorzugsweise zweimal, um nicht gebundenen primären Antikörper zu entfernen und danach mit dem sekundären Antikörper/Enzymkonjugat in verdünnter Lösung behandelt, so dass der Komplex aus primärem Antikörper und sekundärem Antikörper/Enzymkonjugat entsteht. Nach weiteren Waschschritten, wiederum zur Entfernung von nicht oder nur schwach gebundenen Antikörper/Enzymkonjugaten erfolgt die Aufbringung der Farbreaktionslösung, die mit dem Antikörperkomplex unter Bildung eines Farbstoffes reagiert.

Der Auftrag der einzelnen Lösungen kann jeweils mit einem Sprühgerät und/oder einem Kunststoffschwamm erfolgen. Bei sehr stark verschmutzten oder verfärbten Flächen kann durch aufsprühbare Polyacetat-Folie oder einer vergleichbaren Substanz ein Abzug hergestellt werden, der einen Teil des Mikroorganismus (z.B. Pilzmycel) eingebettet hat. Der Nachweis erfolgt mit denselben Verfahrensschritten wie auf der ursprünglichen Oberfläche.

Alternativ kann auch eine Biolumineszenzreaktion zum Nachweis der Mikroorganismen auf der zu untersuchenden Oberfläche eingesetzt werden. Hierbei kann ein die Biolumineszenz katalysierendes Enzym auf den an der zu behandelnden Oberfläche haftenden Mikroorganismus aufgebracht werden, dessen Organismus oder ein Stoffwechselprodukt von diesem mit dem Enzym reagiert, so dass ein Lichtsignal oder eine Farbreaktion ausgelöst wird.

Ein Beispiel einer derartigen Biolumineszenz ist die Reaktion von Luciferasen, die die Oxidation von Luciferin in Gegenwart von Sauerstoff und aus dem Mikroorganismus stammendem ATP zu oxidiertem Luciferin, AMP und einer hohen Quantenausbeute an Licht katalysieren.

Die Art der zu behandelnden Oberflächen, die dem erfindungsgemäßen Verfahren zugänglich sind, ist keineswegs auf die vorstehend beschriebenen beschränkt. So können ebenso Tapeten, Möbeloberflächen, Holzoberflächen, Metalloberflächen oder sonstige Oberflächen, auf denen Mikroorganismen haften bzw. wachsen können, behandelt werden.

## Patentansprüche

1. Verfahren zum Nachweis von Mikroorganismen auf Oberflächen, **dadurch gekennzeichnet, dass** der Mikroorganismus auf einer Oberfläche mit einer Lichtquelle bestrahlt wird, die eine Wellenlänge beinhaltet, die bei Einstrahlung auf den Mikroorganismus, eine Photodetektion des Mikroorganismus zulässt und der Mikroorganismus auf der Oberfläche zusätzlich durch eine chemisch oder immunologisch vermittelte Farb- oder Lichtreaktion nachgewiesen wird.

2. Verfahren nach Anspruch 1, **dadurch gekennzeichnet, dass** die Mikroorganismen Pilze sind.

3. Verfahren nach Anspruch 1, **dadurch gekennzeichnet, dass** die Lichtquelle ultraviolettes Licht beinhaltet.

4. Verfahren nach Anspruch 3, **dadurch gekennzeichnet, dass** die Wellenlänge im Bereich von 210 bis 310 nm liegt.

5. Verfahren nach Anspruch 1, **dadurch gekennzeichnet, dass** die Farbreaktion mit Ninhydrin durchgeführt wird.

6. Verfahren nach Anspruch 5, **dadurch gekennzeichnet, dass** auf die zu untersuchende Fläche eine Ninhydrin-Lösung in einem Lösungsmittel aufgebracht wird.

7. Verfahren nach Anspruch 6, **dadurch gekennzeichnet, dass** als Lösungsmittel leichtflüchtige Lösungsmittel verwendet werden.

8. Verfahren nach Anspruch 6 oder 7, **dadurch gekennzeichnet, dass** die so behandelte Oberfläche auf 70°-100°C erhitzt wird.

9. Verfahren nach Anspruch 8, **dadurch gekennzeichnet, dass** das Erhitzen mit einem Föhn und/oder Heizstrahler erfolgt.

10. Verfahren nach Anspruch 1, **dadurch gekennzeichnet, dass** die Farb- oder Lichtreaktion durch eine Antigen-AntikörperReaktion vermittelt ist.

11. Verfahren nach Anspruch 1 oder 10, **dadurch gekennzeichnet, dass** die Farb- oder Lichtreaktion durch Biolumineszenz vermittelt ist.

12. Verfahren nach einem der Ansprüche 1 bis 9, **dadurch gekennzeichnet, dass** die Farbreaktion durch Reaktion einer chromophoren oder fluorophoren Substanz mit dem Mikroorganismus erfolgt.

13. Verfahren nach Anspruch 1, **dadurch gekennzeichnet, dass** die zu untersuchende Oberfläche mit einem mildwirkenden oberflächenaktiven Mittel gereinigt wird.

14. Verfahren nach Anspruch 1, **dadurch gekennzeichnet, dass** die Oberfläche durch eine Proteinlösung gesättigt wird.

15. Verfahren nach Anspruch 1, **dadurch gekennzeichnet, dass** der Nachweis der Mikroorganismen mittelbar erfolgt, wobei von der zu untersuchenden Oberfläche ein Folienabzug oder Filmabzug hergestellt wird, vorzugsweise unter Verwendung eines Polyacetatfilms.

16. Verfahren nach Anspruch 15, **dadurch gekennzeichnet, dass** der Folienabzug oder Filmabzug durch Sprühen und/oder mit einem Schwamm hergestellt wird.

17. Verfahren nach Anspruch 15, **dadurch gekennzeichnet, dass** die Mikroorganismen auf dieser Folie bzw. auf dem Film nachgewiesen werden.

18. Verfahren nach einem der vorhergehenden Ansprüche, **dadurch gekennzeichnet, dass** die Photodetektion und/oder die Farb- oder Lichtreaktion mit einer Kamera in regelmäßigen oder unregelmäßigen Abständen verfolgt wird.

19. Verfahren nach Anspruch 18, **dadurch gekennzeichnet, dass** die Detektion computerunterstützt durchgeführt wird.

20. Verfahren nach Anspruch 18, **dadurch gekennzeichnet, dass** die Detektion durch Ferndiagnose durchgeführt wird.

21. Verfahren nach einem der vorhergehenden Ansprüche, **dadurch gekennzeichnet, dass** es die folgenden Schritte umfasst:
a) Auftragen einer mit Mikroorganismen reaktiven Substanz auf die zu untersuchende Oberfläche,
b) Durchführen einer Reaktion der Mikroorganismen mit der reaktiven Substanz unter Bildung eines Reaktionsproduktes, und
c) Sichtbarmachen des Reaktionsprodukts.

22. Verfahren nach Anspruch 21, **dadurch gekennzeichnet, dass** die Auswertung durch Sichtinspektion und/oder durch Computerauswertung erfolgt.

23. Verfahren nach Anspruch 1, **dadurch gekennzeichnet, dass** es zur Kontrolle von Filtern eingesetzt wird.

24. Verfahren nach Anspruch 1, **dadurch gekennzeichnet, dass** es zur Kontrolle von Filtern in Klima- oder Lüftungsanlagen eingesetzt wird.

25. Verfahren nach Anspruch 24, **dadurch gekennzeichnet, dass** es in Schutzbelüftungsanlagen, insbesondere von Erdbaumaschinen eingesetzt wird.

26. Verfahren nach Anspruch 23, **dadurch gekennzeichnet, dass** die Filteroberfläche auf der Reinraumseite zusätzlich mit einer chromo- oder fluorophoren Chemikalie behandelt wird.

27. Verfahren nach Anspruch 23, **dadurch gekennzeichnet, dass** die Filteroberfläche auf der Reinraumseite mit Chemikalien imprägniert wird, die nach Biotransformation chromo- oder fluorophor werden.

28. Verfahren nach Anspruch 23, **dadurch gekennzeichnet, dass** die Filterrückseite mit infrarotem, sichtbarem oder ultraviolettem Licht bestrahlt wird.

29. Verfahren nach Anspruch 1, **dadurch gekennzeichnet, dass** es zur Überwachung von Mikroorganismusbewuchs auf Wandoberflächen dient.

30. Verfahren nach Anspruch 29, **dadurch gekennzeichnet, dass** die Wandoberfläche Mauerwerk ist.

31. Verfahren nach Anspruch 29, **dadurch gekennzeichnet, dass** die Wandoberfläche Holz ist.

32. Verfahren nach Anspruch 29, **dadurch gekennzeichnet, dass** die Wandoberfläche eine Metalloberfläche ist.

33. Verfahren nach Anspruch 29, **dadurch gekennzeichnet, dass** die Wandoberfläche eine Tapete ist.

34. Verfahren nach Anspruch 29, **dadurch gekennzeichnet, dass** die Wandoberfläche die Fläche eines Möbelstücks ist.

35. Vorrichtung zur Durchführung des Verfahrens nach Anspruch 1, **dadurch gekennzeichnet, dass** sie folgendes umfasst:
eine Lichtquelle zur Bestrahlung eines Mikroorganismus auf einer zu untersuchenden Oberfläche, die eine Wellenlänge beinhaltet, die bei Einstrahlung auf den Mikroorganismus eine Photodetektion des Mikroorganismus zulässt, und
eine Vorrichtung zum Auftrag eines Reagens auf die Oberfläche, welches eine Farb- oder Lichtreaktion zum Nachweis des Mikroorganismus ermöglicht,
eine Detektionseinrichtung zur Erkennung des Bewuchses des Mikroorganismus, und
eine Diskriminierungseinrichtung zur Bewertung des Grades des Mikroorganismusbewuchses.

36. Vorrichtung nach Anspruch 35, **dadurch gekennzeichnet, dass** die Detektionseinrichtung eine digitale Kamera ist.

37. Vorrichtung nach Anspruch 36, **dadurch gekennzeichnet, dass** die Diskriminierungseinrichtung ein Computer ist. der eine Subtraktion mehrerer von der digitalen Kamera aufgenommener Bilder der zu untersuchenden Oberfläche durchführt.

38. Vorrichtung nach einem der Ansprüche 35 bis 37 **dadurch gekennzeichnet, dass** aufgrund der Berechnung der Diskriminierungseinrichtung ein Warnsignal ausgelöst wird, sofern der Grad des Mikroorganismusbewuchses einen kritischen Grenzwert übersteigt.

39. Vorrichtung nach einem der Ansprüche 35 bis 38, **dadurch gekennzeichnet, dass** die Überwachung durch ein Fernwartungssystem gesteuert wird.

40. Vorrichtung nach Anspruch 39, **dadurch gekennzeichnet, dass** das Fernwartungssystem über Telekommunikationseinrichtungen gesteuert wird.

## Claims

1. Method for detecting microorganisms on surfaces, **characterised in that** the microorganism on a surface is irradiated with a light source including a wavelength which, when irradiated onto the microorganism, permits photodetection of the microorganism, and the microorganism on the surface is additionally detected by a chemically or immunologically mediated colour reaction or light reaction.

2. Method according to claim 1, **characterised in that** the microorganisms are fungi.

3. Method according to claim 1, **characterised in that** the light source includes ultraviolet light.

4. Method according to claim 3, **characterised in that** the wavelength is in the range from 210 to 310 nm.

5. Method according to claim 1, **characterised in that** the colour reaction is carried out with ninhydrin.

6. Method according to claim 5, **characterised in that** a ninhydrin solution in a solvent is applied to the area to be examined.

7. Method according to claim 6, **characterised in that** readily volatile solvents are used as solvent.

8. Method according to claim 6 or 7, **characterised in that** the surface so treated is heated to 70°-100°C.

9. Method according to claim 8, **characterised in that** the heating is effected with a blow dryer and/or a radiant heater.

10. Method according to claim 1, **characterised in that** the colour or light reaction is mediated by an antigen-antibody reaction.

11. Method according to claim 1 or 10, **characterised in that** the colour or light reaction is mediated by bioluminescence.

12. Method according to any one of claims 1 to 9, **characterised in that** the colour reaction is effected by reaction of a chromophoric or fluorophoric substance with the microorganism.

13. Method according to claim 1, **characterised in that** the surface to be examined is cleaned with a mild surfactant.

14. Method according to claim 1, **characterised in that** the surface is saturated by a protein solution.

15. Method according to claim 1, **characterised in that** the detection of the microorganisms takes place indirectly, wherein a film impression replica is made of the surface to be examined, preferably using a polyacetate film.

16. Method according to claim 15, **characterised in that** the film impression replica is made by spraying and/or with a sponge.

17. Method according to claim 15, **characterised in that** the microorganisms are detected on that film.

18. Method according to any one of the preceding claims, **characterised in that** the photodetection and/or the colour or light reaction is tracked with a camera at regular or irregular intervals.

19. Method according to claim 18, **characterised in that** the detection is computer-assisted.

20. Method according to claim 18, **characterised in that** the detection is carried out by remote diagnosis.

21. Method according to any one of the preceding claims, **characterised in that** it comprises the following steps:
a) applying to the surface to be examined a substance that reacts with microorganisms,
b) carrying out a reaction of the microorganisms with the reactive substance to form a reaction product, and
c) visualising the reaction product.

22. Method according to claim 21, **characterised in that** the evaluation is effected by visual inspection and/or by computer evaluation.

23. Method according to claim 1, **characterised in that** it is used for inspecting filters.

24. Method according to claim 1, **characterised in that** it is used for inspecting filters in air-conditioning or ventilation systems.

25. Method according to claim 24, **characterised in that** it is used in protective aeration systems, especially of earth-moving machines.

26. Method according to claim 23, **characterised in that** the filter surface on the clean-room side is treated in addition with a chromophoric or fluorophoric chemical.

27. Method according to claim 23, **characterised in that** the filter surface on the clean-room side is impregnated with chemicals that after biotransformation become chromophoric or fluorophoric.

28. Method according to claim 23, **characterised in that** the rear side of the filter is irradiated with infrared, visible or ultraviolet light.

29. Method according to claim 1, **characterised in that** it serves to monitor microorganism fouling on wall surfaces.

30. Method according to claim 29, **characterised in that** the wall surface is masonry.

31. Method according to claim 29, **characterised in that** the wall surface is wood.

32. Method according to claim 29, **characterised in that** the wall surface is a metal surface.

33. Method according to claim 29, **characterised in that** the wall surface is wallpaper.

34. Method according to claim 29, **characterised in that** the wall surface is the surface of a piece of furniture.

35. Apparatus for carrying out the method according to claim 1, **characterised in that** it comprises the following:
a light source for irradiating a microorganism on a surface to be examined, which light source includes a wavelength which, when irradiated onto the microorganism, permits photodetection of the microorganism, and
an apparatus for applying a reagent to the surface, which reagent renders possible a colour reaction or light reaction for detection of the microorganism,
a detection device for detection of fouling by the microorganism, and
a discrimination device for evaluating the degree of microorganism fouling.

36. Apparatus according to claim 35, **characterised in that** the detection device is a digital camera.

37. Apparatus according to claim 36, **characterised in that** the discrimination device is a computer that carries out subtraction of a plurality of images of the surface to be examined taken by the digital camera.

38. Apparatus according to any one of claims 35 to 37, **characterised in that** on the basis of the calculation of the discrimination device a warning signal is triggered if the degree of microorganism fouling exceeds a critical limit.

39. Apparatus according to any one of claims 35 to 38, **characterised in that** the monitoring operation is controlled by a remote maintenance system.

40. Apparatus according to claim 39, **characterised in that** the remote maintenance system is controlled via telecommunications equipment.

## Revendications

1. Procédé de détection de micro-organismes sur des surfaces, **caractérisé en ce que** le micro-organisme se trouvant une surface est irradié avec une source de lumière ayant une longueur d'onde qui, lors de la projection du rayonnement sur le micro-organisme, permet une photodétection du micro-organisme, et le micro-organisme se trouvant sur la surface est en plus détecté par une réaction produisant une coloration ou une émission de lumière, intervenant par voie chimique ou immunologique.

2. Procédé selon la revendication 1, **caractérisé en ce que** les micro-organismes sont des champignons.

3. Procédé selon la revendication 1, **caractérisé en ce que** la source de lumière contient de la lumière ultraviolette.

4. Procédé selon la revendication 3, **caractérisé en ce que** la longueur d'onde est située dans la fourchette de 210 à 310 nm.

5. Procédé selon la revendication 1, **caractérisé en ce que** la réaction à la couleur est effectuée avec de la ninhydrine.

6. Procédé selon la revendication 5, **caractérisé en ce qu'**une solution de ninhydrine introduite dans un solvant est appliquée sur la surface à examiner.

7. Procédé selon la revendication 6, **caractérisé en ce que** l'on utilise comme solvant un solvant aisément volatil.

8. Procédé selon la revendication 6 ou 7, **caractérisé en ce que** la surface ainsi traitée est chauffée à 70° à 100°C.

9. Procédé selon la revendication 8, **caractérisé en ce que** le chauffage s'effectue avec un fôhn ou un émetteur rayonnant de chauffage.

10. Procédé selon la revendication 1, **caractérisé en ce que** la réaction produisant une coloration ou une émission de lumière est communiquée par une réaction antigène-anticorps.

11. Procédé selon la revendication 1 ou 10, **caractérisé en ce que** la réaction produisant une coloration ou une émission de lumière est communiquée par une biolumescence.

12. Procédé selon l'une des revendications 1 à 9,
**caractérisé en ce que** la réaction à la couleur est effectuée par réaction d'une substance chromophore ou fluorophore avec le micro-organisme.

13. Procédé selon la revendication 1, **caractérisé en ce que** la surface à examiner est nettoyée avec un produit tensio-actif à effet atténuateur.

14. Procédé selon la revendication 1, **caractérisé en ce que** la surface est saturée par une solution de protéine.

15. Procédé selon la revendication 1, **caractérisé en ce que** la détection de micro-organismes est effectuée de façon indirecte, sachant qu'un prélèvement, se présentant sous forme de feuille ou de film, de la surface à examiner est fabriqué, de préférence en utilisant un film en polyacétate.

16. Procédé selon la revendication 15, **caractérisé en ce que** le prélèvement, se présentant sous forme de feuille ou de film, est produit par pulvérisation ou avec une éponge.

17. Procédé selon la revendication 15, **caractérisé en ce que** les micro-organismes, présents sur cette feuille ou sur le film, sont détectés.

18. Procédé selon l'une des revendications 1 à 8,
**caractérisé en ce que** la photodétection et/ou la réaction produisant une coloration ou une émission de lumière est suivie, à des intervalles de temps réguliers ou irréguliers, à l'aide d'une caméra.

19. Procédé selon la revendication 18, **caractérisé en ce que** la détection est effectuée sous l'assistance d'un ordinateur.

20. Procédé selon la revendication 18, **caractérisé en ce que** la détection est effectuée par télédiagnostic.

21. Procédé selon l'une des revendications précédentes, **caractérisé en ce qu'**il comprend les étapes suivantes :
a) application d'une substance, réactive à des microorganismes, sur la surface à examiner,
b) accomplissement d'une réaction des microorganismes avec la substance réactive, avec formation d'un produit de réaction, et
c) mise en évidence du produit de réaction.

22. Procédé selon la revendication 21, **caractérisé en ce que** l'évaluation s'effectue par inspection visuelle et/ou par évaluation par ordinateur.

23. Procédé selon la revendication 1, **caractérisé en ce qu'**il est utilisé pour le contrôle de filtres.

24. Procédé selon la revendication 1, **caractérisé en ce qu'**il est utilisé pour le contrôle de filtres dans des installations de climatisation ou de ventilation.

25. Procédé selon la revendication 24, **caractérisé en ce qu'**il est utilisé dans des installations de ventilation dans un but de protection, en particulier d'engins de terrassement.

26. Procédé selon la revendication 23, **caractérisé en ce que** la surface de filtre située sur le côté salle propre est en plus traitée avec un produit chimique chromophore ou fluorophore.

27. Procédé selon la revendication 23, **caractérisé en ce que** la surface de filtre située sur le côté salle propre est imprégnée de produits chimiques devenant chromophores ou fluorophores après avoir subi une biotransformation.

28. Procédé selon la revendication 23, **caractérisé en ce que** la surface arrière de filtre est exposée à un rayonnement de lumière infrarouge, visible ou ultraviolette.

29. Procédé selon la revendication 1, **caractérisé en ce qu'**il sert à la surveillance de la salissure ou encrassement, par des micro-organismes, sur des surfaces de murs ou parois.

30. Procédé selon la revendication 29, **caractérisé en ce que** la surface de mur ou paroi est une maçonnerie.

31. Procédé selon la revendication 29, **caractérisé en ce que** la surface de mur ou paroi est en bois.

32. Procédé selon la revendication 29, **caractérisé en ce que** la surface de mur ou paroi est une surface métallique.

33. Procédé selon la revendication 29, **caractérisé en ce que** la surface de mur ou paroi est une tapisserie ou du papier-peint.

34. Procédé selon la revendication 29, **caractérisé en ce que** la surface de mur ou paroi est la surface d'un meuble.

35. Dispositif de mise en oeuvre du procédé selon la revendication 1, **caractérisé en ce qu'**il ce qui suit :
- une source de lumière, pour irradier un micro-organisme se trouvant sur une surface à examiner, ayant une longueur d'onde qui, lors de la projection du rayonnement sur le micro-organisme, permet une photodétection du micro-organisme, et un dispositif d'application d'un réactif sur la surface, permettant une réaction produisant une coloration ou une émission de lumière, pour détecter le micro-organisme,
- un dispositif de détection, pour identifier la salissure ou l'encrassement, par de micro-organisme, et
- un dispositif de discrimination, pour évaluer le degré de salissure ou encrassement, par le micro-organisme.

36. Dispositif selon la revendication 35, **caractérisé en ce que** le dispositif de détection est une caméra numérique.

37. Dispositif selon la revendication 36, **caractérisé en ce que** le dispositif de détection est un ordinateur, accomplissant une soustraction de plusieurs images, enregistrées par la caméra numérique, de la surface à examiner.

38. Dispositif selon l'une des revendications 35 à 37, **caractérisé en ce que**, du fait du calcul du dispositif de discrimination, un signal est déclenché dans la mesure où le degré de salissure ou encrassement, par des micro-organismes, dépasse une valeur limite critique.

39. Dispositif selon l'une des revendications 35 à 38, **caractérisé en ce que** la surveillance est commandée par un système de télémaintenance.

40. Dispositif selon la revendication 39, **caractérisé en ce que** le système de télémaintenance est commandé par l'intermédiaire de dispositifs de télécommunication.
